(19) Europäisches Patentamt
European Patent Office
Office européen des brevets

(11) Publication number: **0 277 405 B1**

(12) **EUROPEAN PATENT SPECIFICATION**

(45) Date of publication of patent specification: **13.05.92**

(51) Int. Cl.[5]: **C07C 255/16**, C07C 329/04, C07D 213/643, C07F 9/40, A01N 39/00, A01N 47/06, A01N 57/22

(21) Application number: **87301014.4**

(22) Date of filing: **05.02.87**

(54) **Substituted phenoxypropionaldehyde derivatives.**

(43) Date of publication of application:
**10.08.88 Bulletin 88/32**

(45) Publication of the grant of the patent:
**13.05.92 Bulletin 92/20**

(84) Designated Contracting States:
**AT BE DE ES FR GB IT NL**

(56) References cited:
**EP-A- 0 003 517**
**EP-A- 0 008 973**

(73) Proprietor: **PPG INDUSTRIES, INC.**
**One PPG Place**
**Pittsburgh Pennsylvania 15272(US)**

(72) Inventor: **Raju, Muppala Sarveswara**
**620 Treeside Drive**
**Akron Ohio 44313(US)**

(74) Representative: **Gore, Peter Manson et al**
**W.P. THOMPSON & CO. Coopers Building**
**Church Street**
**Liverpool L1 3AB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.08/2.19/2.0)

## Description

The present invention relates to substituted phenoxypropionaldehyde derivatives, compositions containing such derivatives and the use thereof for pre-emergence or post-emergence control of noxious plants, namely weeds.

According to the present invention there are provided active substituted phenoxypropionaldehyde derivatives represented by the general formula:

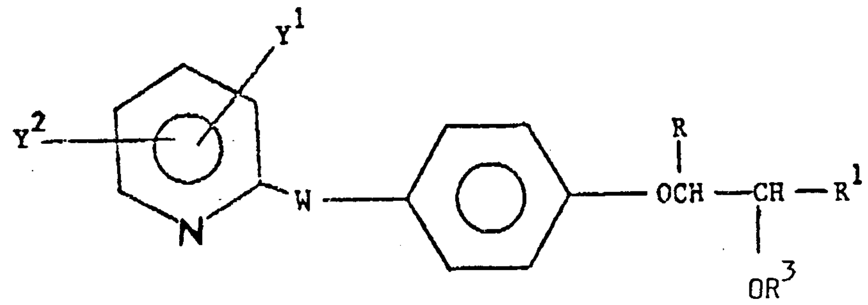

wherein

$Y^1$ and $Y^2$ are independently hydrogen, halogen, nitro, cyano, or a radical of up to 4 carbon atoms selected from alkyl, haloalkyl and alkoxy;

W is O or S;

R is hydrogen or a radical of up to 4 carbon atoms selected from alkyl and alkoxy;

$R^1$ is cyano, vinyl, acetynyl, or dialkylphosphonate;

$R^3$ is hydrogen, a radical of up to 10 carbon atoms selected from alkyl, cycloalkyl, haloalkyl, hydroxyalkyl, oxoalkyl, alkoxyalkyl, alkenyl and alkynyl, or $-COR^6$ or $-CSR^6$ wherein $R^6$ is a radical of up to 10 carbon atoms selected from alkyl, cycloalkyl, haloalkyl, alkoxy, alkenyl, and alkynyl, or amino, $-COOR^7$ or $-COSR^7$ wherein $R^7$ is hydrogen, alkali metal, or a radical of up to 10 carbon atoms selected from alkyl, cycloalkyl, haloalkyl, alkenyl and alkynyl.

Particularly preferred compounds include those represented by the general formula:

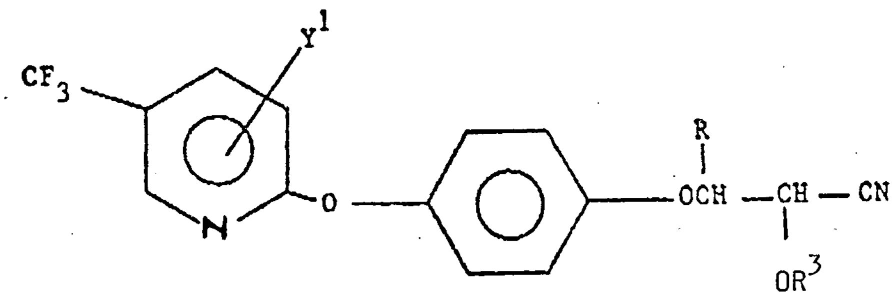

wherein

$Y^1$ is hydrogen or halogen;

R is an alkyl radical of up to 4 carbon atoms; and

$R^3$ is hydrogen, a radical of up to 10 carbon atoms selected from alkyl and haloalkyl, or $-COR^6$ or $-CSR^6$ wherein $R^6$ is a radical of up to 10 carbon atoms selected from alkyl, and alkoxy, or $-COOR^7$ or $-COSR^7$ wherein $R^7$ is hydrogen, alkali metal or an alkyl radical of up to 10 carbon atoms.

The group $Y^1$ is preferably in the 2-position. Group $Y^1$ is also preferably chlorine when it is halogen. Preferably R is methyl.

The compounds of the present invention may be prepared using techniques and starting materials known and available to those skilled in the art, and preparation of certain of the compounds of the present invention are illustrated by the following Examples, wherein a compound having the following formula is prepared:

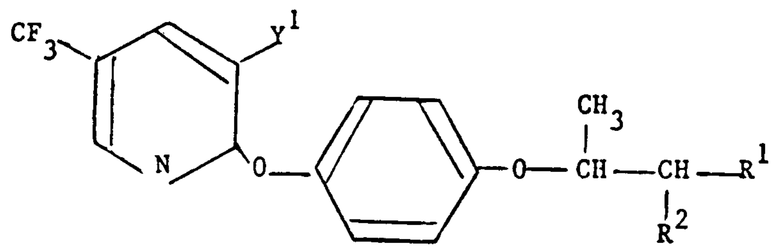

wherein $Y^1$, $R^1$ and $R^2$ are as follows:

| Example | $Y^1$ | $R^1$ | $R^2$ |
|---|---|---|---|
| I | -H | -CN | $-O-COO-C_2H_5$ |

Example I

Preparation of: 2-ethoxycarbonyloxy-3-[4-(5-trifluoromethyl-2-pyridyloxy)phenoxy]butyronitrile.

A mixture of 0.28 grams of 3-[4-(5-trifluromethyl-2-pyridyloxy) phenoxy]propionaldehyde, 1.0 gram of powdered sodium cyanide, 0.1 gram of tetrabetyl ammonium hydrogen sulphate, 3.2 millilitre of ethyl chlorocarbonate and 40 milliliters of methylene chloride was stirred overnight at room temperature under a nitrogen blanket. The reaction mixture was then diluted with 50 milliliters of methylene chloride and washed consecutively with 2 x 40 milliliter portions of water, 2 x 40 milliliter portions of saturated aqueous sodium bicarbonate and 2 x 40 milliliter portions of water. The organic phase was dried over anhydrous sodium sulfate and evaporated affording about 0.42 gram of viscous, gummy residue. The residue was eluted over silica gel using methylene chloride as the eluent, 50 milliliter fractions being collected and analyzed by TLC. Fractions 5 to 11 were combined and evaporated affording about 0.3 gram of colorless viscous material confirmed by NMR and MS analyses as the desired product.

Examples II to V

Following the procedure described in the foregoing Example using 3-[4-(5-trifluoromethyl-2-pyridyloxy)-phenoxy]-2-hydroxy butyronitrile (prepared in situ) as a starting material, the following compounds were also prepared:

II. The compound, 2-phenoxycarbonyl-3-[4-(5-trifluoromethyl-2-pyridyloxy)phenoxy]butyronitrile, by reaction of said starting material with phenyl chloroformate.

III. The compound, 2-acetyloxy-3-[4-(-trifluoromethyl-2-pyridyloxy)phenoxy]butyronitrile, by reaction of said starting material with acetylchloride.

IV. The compound, 2-methyloxycarbonyloxy-3-[4-(5-trifluoromethyl-2-pyridyloxy)phenoxy]butyronitrile, by reaction of said starting material with methylchloroformate.

V. The compound, 2-allyloxycarbonyloxy-3-[4-(5-trifluormethyl-2-pyridyloxy)phenoxy]butyronitrile, by reaction of said starting material with allyl chloroformate.

Weed control in accordance with this invention is effected by applying to the soil prior to emergence of weeds therefrom or to the plant surfaces subsequent to emergence from the soil, a herbicidally effective amount of a compound of this invention. It is, of course, to be understood that the term "a compound of this invention" also includes mixtures of such compounds or a formulation containing a compound or mixture of compounds of this invention.

The term "herbicidally effective amount" is that amount of a compound of this invention required to so injure or damage weeds such that the weeds are incapable of recovering following application while not causing substantial injury to any valuable crop amongst which the weeds might be growing. The quantity of

a compound of this invention applied in order to exhibit a satisfactory herbicidal effect may vary over a wide range and depends on a variety of factors, such as, for example, hardiness of a particular weed species, extent of weed infestation, climatic conditions, soil conditions, method of application, and the like. Typically, 11.2 or less grams/are (one or less pound per acre) of a compound of this invention would be expected to provide satisfactory weed control, although in some instances application rates in excess of one pound per acre, e.g., up to 22.4 or more gms/are (up to 2 or more pounds per acre) might be required. Of course, the efficacy of a particular compound against a particular weed species may readily be determined by routine laboratory or field testing in a manner well known to the art. It is expected that satisfactory weed control can be had at a rate of application in the range of 1.1 to 11.2 gms/are (0.1 to 1.0 pound per acre).

Of course, a compound of this invention can be formulated according to routine methods with any of several known and commonly used herbicidal diluents, adjuvants and carriers. The formulations can contain liquid carriers and adjuvants such as organic solvents, as well as compounds such as, for example, emulsifiers, stabilizers, dispersants, suspending agents, spreaders, penetrants and wetting agents. Typical carriers utilized in dry formulations include clay, talc, diatomaceous earth and silica. Preferred formulations are those in the form of wettable powders, flowables, dispersible granulates or aqueous emulsifiable concentrates which can be diluted with water at the site of application. Also, dry formulations such as granules, dusts, and the like, may be used.

When desired, a compound of this invention can be applied in combination with other herbicidal agents in an effort to achieve even broader vegetative control. Typical herbicides which can be conveniently combined with one or more compounds of the present invention include atrazine, hexazinone, metribuzin, ametryn, cyanazine, cyprazine, prometon, prometryn, propazine, simazine, terbutryn, propham, alachlor, acifluorfen, bentazon, metolachlor and N,N-dialkyl thiocarbamates such as EPTC, butylate or vernolate. These, as well as other herbicides described, for example, in the Herbicide Handbook of the Weed Science Society of America, may be used in combination with a compound or compounds of the invention. Typically such formulations will contain from about 5 to about 95 percent by weight of a compound of this invention.

The herbicidal formulations contemplated herein can be applied by any of several methods known to the art. Generally, the formulation will be applied as an aqueous spray. Such application can be carried out by conventional ground equipment, or if desired, the sprays can be aerially applied. Soil incorporation of such surface applied herbicides is accomplished by natural leaching, and is of course facilitated by natural rainfall and melting snow. If desired, however, the herbicides can be incorporated into the soil by conventional tillage means.

Compounds of this invention have been found especially useful for controlling grassy weeds but could be used for preemergence and postemergence control of a wide variety of grassy weeds. Typical of the various species of vegetative growth that may be controlled, combated, or eliminated are, for example, annuals such as foxtail, crabgrass, field pennycress, ryegrass, goose grass, wild oats, barnyardgrass, hemp nettle, spurge, pondweed, cheatgrass, fall panicum, witchgrass, watergrass, and similar annual grasses and weeds. Biennials that may be controlled include wild barley and campion. Also controlled by the compounds of this invention may be perennials such as quackgrass, Johnsongrass, horsetail and cattail.

The compound prepared as described in the Examples were individually tested for herbicidal efficacy, against a variety of grassy weed species, under controlled laboratory conditions of light, humidity and temperature. A solvent solution of each compound was applied, both preemergence and postemergence, to test flats containing the various weed species, and herbicidal efficacy was determined by periodic visual inspection, after application of the compounds vis a vis an untreated control. Herbicidal efficacy was determined on a Numerical Injury Rating (NIR) scale of from 0 (no injury) to 10 (all plants dead). A NIR rating of 7 to 9 indicates severe injury; a NIR rating of 4 to 6 indicates moderate injury, that is, plant growth is reduced to the extent that normal growth could be expected, but only under ideal conditions; and a NIR rating of 1 to 3 indicates slight injury.

The following Tables give the individual and average pre-emergence (Table 1) and post-emergence (Table II) NIR determined for the compound prepared as described in Example I on the grassy (GR) weed species to which the compounds were applied, at the indicated rate of application in pounds per acre. The NIR was determined three weeks subsequent to application.

Table I

| Compd: | I |
|---|---|
| YLFX | 10 |
| CBGS | 10 |
| JNGS | 10 |
| WOAT | 3 |
| BNGS | 10 |
| Rate | 0.5 |
| Average | 8.6 |

Table II

| Compd: | I |
|---|---|
| YLFX | 8 |
| JNGS | 7 |
| WOAT | 1 |
| BNGS | 9 |
| Rate | 1.1 |
| Average | 6.3 |

The grassy weeds used in the screening tests were barnyardgrass (BNGS), large crabgrass (CBGS), Johnsongrass (JNGS), wild oats (WOAT) and yellow foxtail (YLFX).

**Claims**

**1.** A compound having the general formula:

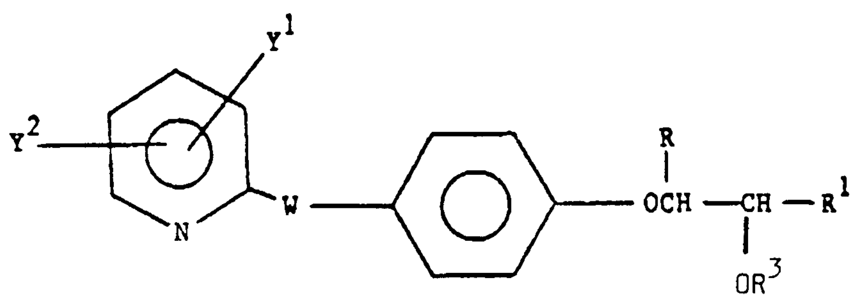

wherein

$Y^1$ and $Y^2$ are independently hydrogen, halogen, nitro, cyano, or a radical of up to 4 carbon atoms selected from alkyl, haloalkyl and alkoxy;

W is O or S;

R is hydrogen or a radical of up to 4 carbon atoms selected from alkyl and alkoxy;

$R^1$ is cyano, vinyl, acetynyl or dialkylphosphonate;

$R^3$ is hydrogen, a radical of up to 10 carbon atoms selected from alkyl, cycloalkyl, haloalkyl, hydroxyalkyl, oxoalkyl, alkoxyalkyl, alkenyl and alkynyl, or $-COR^6$ or $-CSR^6$ wherein $R^6$ is a radical of up to 10 carbon atoms selected from alkyl, cycloalkyl, haloalkyl, alkoxy, alkenyl, and alkynyl, or amino, $-COOR^7$ or $-COSR^7$ wherein $R^7$ is hydrogen, alkali metal, or a radical of up to 10 carbon atoms selected from alkyl, cycloalkyl, haloalkyl, alkenyl and alkynyl.

2. A compound according to claim 1, having the general formula:

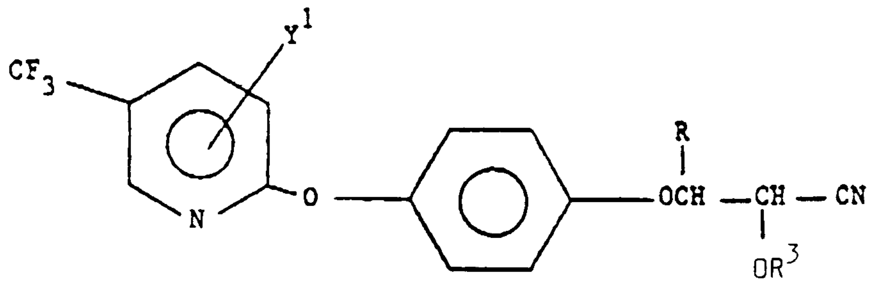

wherein

$Y^1$ is hydrogen or halogen;

R is an alkyl radical of up to 4 carbon atoms; and

$R^3$ is hydrogen, a radical of up to 10 carbon atoms selected from alkyl and haloalkyl, or $-COR^6$ or $-CSR^6$ wherein $R^6$ is a radical of up to 10 carbon atoms selected from alkyl, and alkoxy, or $-COOR^7$ or $-COSR^7$ wherein $R^7$ is hydrogen, alkali metal or an alkyl radical of up to 10 carbon atoms.

3. A compound according to claim 2, wherein the group $Y^1$ is in the 2-position.

4. A compound according to any of claims 1 to 3, wherein the group $Y^1$ is chlorine.

5. A herbicidal composition containing an agronomically acceptable carrier and a herbicidally effective amount of a compound or mixture of compounds according to any of claims 1 to 4.

6. A herbicidal composition according to claim 5, which is in the form of a wettable powder, a flowable, a dispersible granulate or an aqueous emulsifiable concentrate which can be diluted with water at a desired site of application.

7. A herbicidal composition according to claim 5 or 6, in which the compound or mixture of compounds according to any of claims 1 to 4 is used together with one or more other herbicidal agents.

8. A herbicidal composition according to claim 7, which contains 5 to 95 per cent by weight of a compound or mixture of compounds according to any of claims 1 to 4.

9. A method of controlling weeds wherein a herbicidally effective amount of herbicide is applied to a growth medium prior to emergence of weeds therefrom or to the weeds subsequent to emergence from the growth medium, the herbicide comprising a compound or mixture of compounds according to any of claims 1 to 4 or being in the form of a composition according to any of claims 5 to 8.

**Revendications**

**1.** Composé de formule générale suivante:

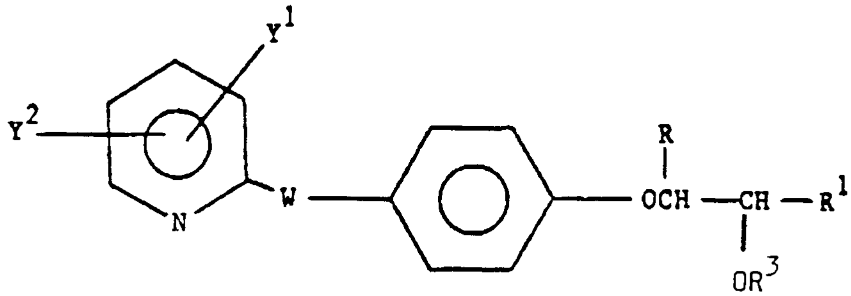

dans laquelle

$Y^1$ et $Y^2$ représentent indépendamment l'atome d'hydrogène, un halogène, le groupe nitro ou cyano, ou un radical ayant jusqu'à 4 atomes de carbone choisi parmi les groupes alkyle, halogénoalkyle et alkoxy;

W représente O ou S;

R représente l'atome d'hydrogène ou un radical ayant jusqu'à 4 atomes de carbone choisi parmi les groupes alkyle et alkoxy;

$R^1$ représente le radical cyano, vinyle, acétynyle ou dialkylphosphonate;

$R^3$ représente l'atome d'hydrogène, un radical comprenant jusqu'à 10 atomes de carbone choisi parmi les groupes alkyle, cycloalkyle, halogénoalkyle, hydroxyalkyle, oxoalkyle, alkoxyalkyle, alkényle et alkynyle, ou $-COR^6$ ou $-CSR^6$, dans lequel $R^6$ est un radical comprenant jusqu'à 10 atomes de carbone choisi parmi les radicaux alkyle, cycloalkyle, halogénoalkyle, alkoxy, alkényle, et alkynyle, ou un groupe amino, $-COOR^7$ ou $-COSR^7$, dans lequel $R^7$ est un atome d'hydrogène, un métal alcalin, ou un radical comprenant jusqu'à 10 atomes de carbone choisi parmi les groupes alkyle, cycloalkyle, halogénoalkyle, alkényle et alkynyle.

**2.** Composé selon la revendication 1 de formule générale suivante:

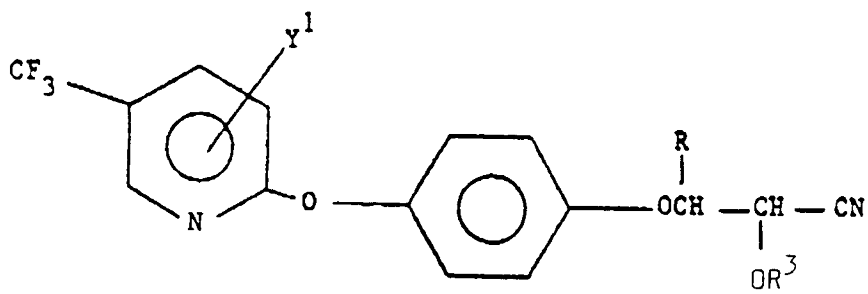

dans laquelle

$Y^1$ représente l'atome d'hydrogène ou un halogène;

R représente un radical alkyle ayant jusqu'à 4 atomes de carbone; et

$R^3$ représente l'atome d'hydrogène, un radical comprenant jusqu'à 10 atomes de carbone choisi parmi les groupes alkyle, halogéno-alkyle, ou $-COR^6$ ou $-CSR^6$ dans lequel $R^6$ est un radical comprenant jusqu'à 10 atomes de carbone choisi parmi les radicaux alkyle et alkoxy, ou $-COOR^7$ ou $-COSR^7$ dans lequel $R^7$ est un atome d'hydrogène, un métal alcalin, ou un radical alkyle comprenant jusqu'à 10 atomes de carbone.

**3.** Composé selon la revendication 2 dans lequel le groupe $Y^1$ se trouve en position 2.

**4.** Composé selon l'une des revendications 1 à 3 dans lequel le groupe $Y^1$ est l'atome de chlore.

5. Composition herbicide contenant un véhicule acceptable du point de vue agronomique et une quantité efficace du point de vue herbicide d'un composé ou d'un mélange de composés selon l'une des revendications 1 à 4.

6. Composition herbicide selon la revendication 5, qui se présente sous la forme de poudre mouillable, de poudre fluide, de granulés dispersibles ou de concentré aqueux émulsifiable que l'on peut diluer dans l'eau sur le site d'application désiré.

7. Composition herbicide selon la revendication 5 ou 6, dans laquelle le composé ou le mélange de composés selon l'une des revendications 1 à 4 est utilisé en association avec un ou plusieurs autres agents herbicides.

8. Composition herbicide selon la revendication 7, qui contient 5 à 95 pour cent en poids d'un composé ou d'un mélange de composés selon l'une des revendications 1 à 4.

9. Méthode d'élimination des mauvaises herbes dans laquelle on applique une quantité efficace du point de vue herbicide d'un herbicide au milieu de croissance avant l'émergence des mauvaises herbes ou aux mauvaises herbes après leur émergence du milieu de culture, l'herbicide comprenant un composé ou un mélange de composés selon l'une des revendications 1 à 4 et se présentant sous la forme d'une composition selon l'une des revendications 5 à 8.

**Patentansprüche**

1. Verbindung mit der allgemeinen Formel:

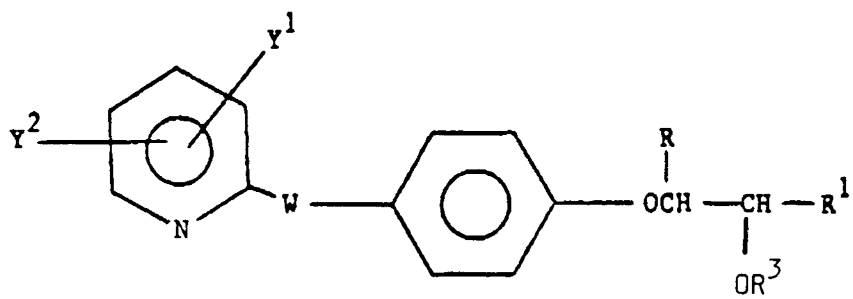

wobei $Y^1$ und $Y^2$ unabhängig Wasserstoff, Halogen, Nitro, Cyano oder ein Rest mit bis zu 4 Kohlenstoffatomen, ausgewählt aus Alkyl, Haloalkyl und Alkoxy, bedeuten; W für O oder S steht; R Wasserstoff oder einen Rest mit bis zu 4 Kohlenstoffatomen, ausgewählt aus Alkyl und Alkoxy, bedeutet;
$R^1$ für Cyano, Vinyl, Acetinyl oder Dialkylphosphonat steht; $R^3$ Wasserstoff, einen Rest mit bis zu 10 Kohlenstoffatomen, ausgewählt aus Alkyl, Cycloalkyl, Haloalkyl, Hydroxyalkyl, Oxoalkyl, Alkoxyalkyl, Alkenyl und Alkinyl, oder $-COR^6$ oder $-CSR^6$, wobei $R^6$ ein Rest mit bis zu 10 Kohlenstoffatomen, ausgewählt aus Alkyl, Cycloalkyl, Haloalkyl, Alkoxy, Alkenyl und Alkinyl ist, oder Amino, $-COOR^7$ und $-COSR^7$ bedeutet, wobei $R^7$ Wasserstoff, Alkalimetall oder ein Rest mit bis zu 10 Kohlenstoffatomen, ausgewählt aus Alkyl, Cycloalkyl, Haloalkyl, Alkenyl oder Alkinyl, ist.

**2.** Verbindung gemäß Anspruch 1 mit der allgemeinen Formel:

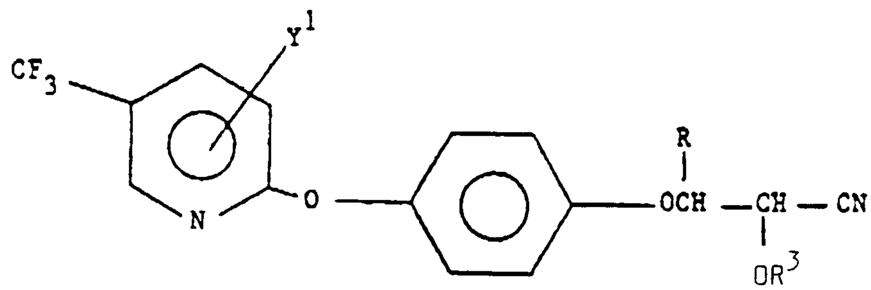

wobei $Y^1$ für Wasserstoff oder Halogen steht; R ein Alkylrest mit bis zu 4 Kohlenstoffatomen ist; und $R^3$ Wasserstoff, einen Rest mit bis zu 10 Kohlenstoffatomen, ausgewählt aus Alkyl und Haloalkyl, oder $-COR^6$ oder $-CSR^6$, wobei $R^6$ ein Rest mit bis zu 10 Kohlenstoffatomen, ausgewählt aus Alkyl und Alkoxy, ist, oder $-COOR^7$ oder $-COSR^7$ bedeutet, wobei $R^7$ Wasserstoff, Alkalimetall oder ein Alkylrest mit bis zu 10 Kohlenstoffatomen ist.

**3.** Verbindung gemäß Anspruch 2, wobei die Gruppe $Y^1$ in der 2-Position steht.

**4.** Verbindung gemäß einem der Ansprüche 1 bis 3, wobei die Gruppe $Y^1$ Chlor ist.

**5.** Heribzide Zusammensetzung, enthaltend einen agronomisch akzeptablen Träger und eine herbizid wirksame Menge einer Verbindung oder Mischung von Verbindungen gemäß einem der Ansprüche 1 bis 4.

**6.** Herbizide Zusammensetzung gemäß Anspruch 5, welche in der Form eines benetzbaren Pulvers, eines fließfähigen, eines dispergierbaren Granulats oder eines wässrigen emulgierbaren Konzentrats vorliegt, das an einer gewünschten Applikationsstelle mit Wasser verdünnt werden kann.

**7.** Herbizide Zusammensetzung gemäß Anspruch 5 oder 6, in der die Verbindung oder Mischung von Verbindungen gemäß einem der Ansprüche 1 bis 4 zusammen mit einem oder mehreren anderen herbiziden Mitteln verwendet wird.

**8.** Herbizide Zusammensetzung gemäß Anspruch 7, enthaltend 5 bis 95 Gew.-% einer Verbindung oder Mischung von Verbindungen gemäß einem der Ansprüche 1 bis 4.

**9.** Verfahren zum Bekämpfen von Unkräutern, wobei eine herbizid wirksame Menge des Herbizids vor dem Auflaufen der Unkräuter auf ein Wuchsmedium appliziert wird oder auf die Unkräuter appliziert wird, nachdem diese aus dem Wuchsmedium herausgewachsen sind, wobei das Herbizid eine Verbindung oder Mischung von Verbindungen gemäß einem der Ansprüche 1 bis 4 umfaßt oder in der Form einer Zusammensetzung gemäß einem der Ansprüche 5 bis 8 vorliegt.